# EUROPEAN PATENT SPECIFICATION

(11) **EP 3 010 926 B1**
(45) Date of publication and mention of the grant of the patent: **20.11.2019**
(21) Application number: 14732980.9
(22) Date of filing: 18.06.2014
(51) Int. Cl.: C07D 498/18, C07C 303/28, A61K 31/436, C07F 7/18

(54) **ALKYLATION WITH AN ALKYL FLUOROALKYL SULFONATE**
ALKYLIERUNG MIT EINEM ALKYL FLUOROALKYLSULFONAT
ALKYLATION AU MOYEN D'UN FLUOROALKYLSULPHONATE D'ALKYLE

(30) Priority: 20.06.2013 US 201361837224 P
(43) Date of publication of application: 27.04.2016
(73) Proprietor: Novartis AG, 4056 Basel (CH)
(72) Inventor: ACEMOGLU, Murat, CH-4002 Basel (CH); VEITCH, Gemma, CH-4002 Basel (CH)
(74) Representative: Kristl, Jernej
(86) International application number: PCT/IB2014/062375
(87) International publication number: WO 2014/203185

(56) References cited:
- WO-A1-2012/103959
- US-A1- 2009 292 118
- SOONG HOON KIM ET AL: "Concise Synthesis of the Calicheamicin Oligosaccharide Using the Sulfoxide Glycosylation Method", JOURNAL OF THE AMERICAN CHEMICAL SOCIETY, vol. 116, no. 5, 1 March 1994 (1994-03-01) , pages 1766-1775, XP055141224, ISSN: 0002-7863, DOI: 10.1021/ja00084a018
- YOSHIDA T ET AL: "A LARGE-SCALE PREPARATION OF (3S,4S)-3-(TERT-BUTOXYCARBONYL)AMINO- 4-METHYLPYRROLIDINE AND ITS ANALOGS FROM L-ASPARTIC ACID", CHEMICAL AND PHARMACEUTICAL BULLETIN, PHARMACEUTICAL SOCIETY OF JAPAN, JP, vol. 44, no. 5, 1 May 1996 (1996-05-01), pages 1128-1131, XP002059605, ISSN: 0009-2363
- DATABASE WPI Section Ch, Week 201166 Thomson Scientific, London, GB; Class B02, AN 2011-K34323 XP002729922, -& CN 102 127 092 A (UNIV SOUTHEAST) 20 July 2011 (2011-07-20)

## Description

### Field of the Invention

The present invention relates in general to the field of chemical technology and in particular to a process for preparing an active pharmaceutical ingredient (API) or intermediates thereof by using an alkyl fluoroalkyl sulfonate. Particularly, the present invention relates to a process for preparing a chemical compound comprising a step of reacting a nucleophile with an alkyl fluoroalkyl sulfonate in the presence of a base. The invention also relates to a process for preparing an alkyl fluoroalkyl sulfonate. The invention further relates to the use of a base in a chemical reaction comprising an alkyl fluoroalkyl sulfonate. The processes and uses described herein are suitable for preparing chemical compounds, reactants or intermediates thereof, and in particular for preparing API or reactants, like for example everolimus and reactants or reagents used in its preparation.

### Background of the Invention

Sulfonate esters are useful in synthetic organic chemistry as they are highly reactive towards nucleophiles in substitution reactions. An example of an excellent sulfonate leaving group is the trifluoromethanesulfonate group, in which the extremely electronegative fluorine atoms cause the anionic leaving group to be especially stable. In principle any fluorinated alkylsulfonate, for example trifluoroethylsulfonate, nonafluorobutylsulfonate or the like, could be applied as highly reactive leaving groups as well.

The trifluoromethanesulfonate group (CF₃SO₃⁻; named also triflate) is particularly valuable in synthetic organic chemistry due to its ability to function both as an inert anion and a very good leaving group. Soong Hoon Kim et al. (J. Am. Chem. Soc. 1994, 116, 1766) disclosed the use of a triflate in the synthesis of the Calicheamicin Oligosaccharide. Relatively good accessibility, safety and stability of the anion add to the usefulness of the trifluoromethanesulfonate group as a leaving group in synthetic organic chemistry. A commonly used reagent to introduce the trifluoromethanesulfonate group onto an alcohol is the highly reactive triflic anhydride ((CF₃SO₂)₂O), which can be prepared by P₂O₅-mediated dehydration of trifluoromethanesulfonic acid (Hendrickson J. B., Sternbach D. D., Bair K. W., Acc Chem Res, 1977, 10, 306).

Alkyl fluoroalkyl sulfonate, particularly trifluoromethanesulfonate (known also under the name triflate), can be prepared by using various procedures. For example, alkyl fluoroalkyl sulfonate can be obtained by reacting an alcohol with the appropriate fluoroalkylsulfonic acid anhydride (Hendrickson J. B., Sternbach D. D., Bair K. W., Acc Chem Res, 1977, 10, 306 and also Yoshida et al. Chem. Pharm. Bull. 1996, 44(5), 1128).

Alkyl fluoroalkyl sulfonate can also be utilized in the synthesis of chemical compounds acting as active pharmaceutical ingredients. To name just two, Everolimus and Umirolimus (trade name Biolimus®) can be prepared by applying fluoroalkyl sulfonate chemistry. Everolimus (RAD-001), 40-O-(2-hydroxy)-ethyl-rapamycin of formula (1), is a synthetic derivative of rapamycin (sirolimus), which is a known macrolide antibiotic produced by Streptomyces hygroscopicus. Everolimus is an immunosuppressive and anticancer drug that inhibits intracellular mTOR ("mammalian Target of rapamycin"). It exerts its effect via interaction with intracellular receptor FKBP12 (FK506-binding protein 12) and by modulating translation of specific mRNAs. Everolimus is marketed by Novartis as a transplantation medicine under the tradenames Zortress® (USA) and Certican® (outside USA) and in oncology as Afinitor®.

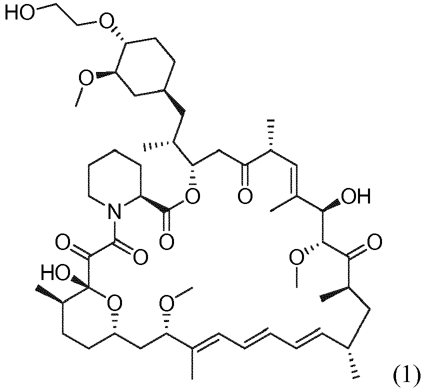

WO2012/066502 discloses a process for preparing everolimus by reacting sirolimus (rapamycin) with either 4 or 8 equivalents of 2-(-t-butyldimethylsilyl)oxyethyl triflate in dichloromethane in the presence of a base which is preferably 2,6-lutidine, followed by cleavage of the t-butyldimethylsilyl protecting group. The overall yield for the two steps is 45% at best. There are many examples where the yield is much lower. For example, when toluene is used as the solvent for the alkylation step, the yield of the 2-step reaction from rapamycin to everolimus drops to below 30%. When ethyl acetate is used as the solvent for the alkylation step the yield drops to around 30%. WO2012/103959 discloses a process for preparing everolimus that is based on a reaction of rapamycin with 2-(t-hexyldimethylsilyloxy)ethyl triflate in an inert solvent in the presence of a base such as 2,6-lutidine, tris(2-methylpropyl)amine, or N,N-diisopropylethylamine. The resultant silylated intermediate can then undergo a silyl group cleavage to afford everolimus. The only experimental example described in the document shows conversion of rapamycin to everolimus in an overall yield of 53%: Here, rapamycin is reacted with 4 equivalents of the preformed 2-(t-hexyldimethylsilyloxy)ethyl triflate (triflate was prepared by using tris(2-methylpropyl)amine, as base) in the presence of N,N-diisopropylethylamine in a solvent mixture comprising 15% of 1,2-dimethoxyethane and 85% toluene at 70 °C. One drawback of this reaction is the lower stability of triflate esters and also of Rapamycin at higher temperatures. An additional disadvantage is the use of 1,2-dimethoxyethane, which is a solvent of environmental concern. The intermediate thus formed then undergoes silyl group cleavage with hydrochloric acid in methanol. The article in the Journal of Labelled Compounds and Radiopharmaceuticals, 42, 29-41 (1999) discloses a mono-tert-butyldiphenylsilyl protecting group in the synthesis of everolimus. US2005/192311 A1 and US2009/292118 A1 disclose a process for preparing umirolimus (40-O-[(2'-ethoxy)ethyl]-rapamycin). Umirolimus is prepared from rapamycin (sirolimus) via an alkylation reaction by reacting a triflate reagent in the presence of N,N-diisopropylethylamine (yield of the 1 alkylation step is in the range of 30-45%).

It is an object of the present invention to provide a new alkylation process that allows use of an alkyl fluoroalkyl sulfonate in preparing a chemical compound in a shorter time, higher yield and in an improved, more economic and simplified fashion. Specific aspects of the present invention can be applied for preparing everolimus or reagents involved in its preparation.

### Summary of the Invention

Since the fluoroalkylsulfonate groups, particularly the trifluoromethanesulfonate, trifluoroethylsulfonate and nonafluorobutylsulfonate, are excellent leaving groups, the in-situ degradation of reactants containing this functionality in the presence of a base represents a potential problem for their application. Decomposition of reactants such as fluoroalkyl sulfonates, e.g. alkyl trifluoromethanesulfonate, reduces process yields, or, increases the costs of the process when excess of the alkyl fluoroalkyl sulfonate reagent, e.g. alkyl trifluoromethanesulfonate reagent, is required. Such degradation can occur for example by quaternization of the tertiary amine base which is commonly employed in the reaction or by elimination of the sulfonate leaving group. The degradation and side reaction problems are characteristic of nucleophilic substitution reactions that require longer reaction times. One example of such a reaction is the alkylation of hindered alcohols. The side reactions in the reaction mixture can begin instantly and the longer the reaction runs, the greater the effect on the reaction efficiency and yield. For example, the problem is particularly evident when the reaction takes more than 1 hour, more than 2 hours, more than 10 hours, especially more than 18 hours, for example more than 24 hours to complete.

To solve the aforementioned object, the present disclosure provides a process according to claim 1. The present invention further provides a process according to claim 5 and use of a specifically defined base in a reaction comprising an alkyl fluoroalkyl sulfonate according to claim 9. Preferred embodiments are set forth in the subclaims.

Surprisingly it has been found that in a process for preparing a chemical compound, where rapamycin is reacted with an alkyl fluoroalkyl sulfonate in the presence of a base, the base of formula NR1R2R3:
- R1 and R2 are independently 2-methylpropyl or isopropyl; and
- R3 is -CH(R4)(R5), wherein R4 and R5 are identical or different alkyls that are optionally connected to form a ring;
exhibits significantly reduced reactivity towards the alkyl fluoroalkyl sulfonate reagent. Choosing the base of the abovementioned formula prevents unnecessary decomposition of the fluoroalkyl sulfonate reagents in the reaction mixture, reduces the need for a large excess of alkyl fluoroalkyl sulfonate reagent in the reaction mixture, increases the reaction economy and yields and makes the process cheaper. In addition, by safeguarding the stability of the alkyl fluoroalkyl sulfonate reagent with the help of the base, the reaction itself is cleaner which makes the subsequent purification steps easier and more efficient. Choosing the base in combination with a certain protecting group may further enhance the process efficiency.

It came as a surprise that increasing the steric hindrance of the more commonly used bases such as N,N-Diisopropyl-ethylamine and tris(2-methylpropyl)amine would make such a difference to the stability of the alkyl fluoroalkyl sulfonate in a process where a nucleophile is reacted with the alkyl fluoroalkyl sulfonate in the presence of a base. The hindered bases described herein are not available commercially and have therefore received little attention. Particularly the preferred base used according to the present invention would not be perceived as the base of choice in the context of preventing side reactions as shown here.

In the first aspect, the present invention provides a process for preparing a chemical compound comprising reacting rapamycin with an alkyl fluoroalkyl sulfonate, wherein the alkyl part of the alkyl fluoroalkyl sulfonate can be further substituted; in the presence of a base, wherein the base is of formula NR1R2R3:
- R1 and R2 are independently 2-methylpropyl or isopropyl; and
- R3 is -CH(R4)(R5), wherein R4 and R5 are identical or different alkyls that are optionally connected to form a ring, and wherein the alkyl is a straight or branched carbon chain of C2-C10-alkyl.

In the second aspect, the present invention provides a process for preparing an alkyl fluoroalkyl sulfonate by reacting an alcohol with a fluoroalkylsulfonic acid anhydride in the presence of a base, wherein the base is of formula NR1R2R3:
- R1 and R2 are independently 2-methylpropyl or isopropyl; and
- R3 is -CH(R4)(R5), wherein R4 and R5 are identical or different alkyl that are optionally connected to form a ring, and wherein the alkyl is a straight or branched carbon chain of C2-C10-alkyl,

In the third aspect, the present invention provides use of a base having formula NR1R2R3 in preparing everolimus, wherein:
- R1 and R2 are independently 2-methylpropyl or isopropyl; and
- R3 is -CH(R4)(R5), wherein R4 and R5 are identical or different alkyls that are optionally connected to form a ring;
in a chemical reaction comprising an alkyl fluoroalkyl sulfonate, and wherein the alkyl is a straight or branched carbon chain of C2-C10-alkyl.

Aspects, advantageous features and preferred embodiments of the present disclosure summarized in the following items, respectively alone or in combination, are also disclosed herein.
1. A process for preparing a chemical compound comprising reacting rapamycin with an alkyl fluoroalkyl sulfonate in the presence of a base, wherein the base is of formula NR1R2R3:
   - R1 and R2 are independently 2-methylpropyl or isopropyl; and
   - R3 is -CH(R4)(R5), wherein R4 and R5 are identical or different alkyls that are optionally connected to form a ring.
2. A process for preparing a chemical compound comprising reacting rapamycin with an alkyl fluoroalkyl sulfonate according to item 1, wherein the fluoroalkyl sulfonate part of the alkyl fluoroalkyl sulfonate comprises C1-C4 alkyl substituted by at least one fluoride, preferably the alkyl fluoroalkyl sulfonate being alkyl trifluoromethylsulfonate, alkyl trifluoroethylsulfonate or alkyl nonafluorobutylsulfonate, especially is alkyl trifluoromethanesulfonate.
3. The process for preparing a chemical compound according to item 1 or 2, wherein the alkyl part of the alkyl fluoroalkyl sulfonate is substituted with a functional group, wherein the functional group is protected with a protecting group.
4. The process for preparing a chemical compound according to any one of items 1 to 3, wherein the alkyl fluoroalkyl sulfonate is a compound of formula (2): 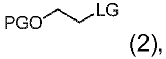 wherein
   - PG is protecting group; and
   - LG is fluoroalkyl sulfonate.
5. The process for preparing a chemical compound according to any one of items 1 to 4, wherein the fluoroalkyl sulfonate part of the alkyl fluoroalkyl sulfonate is trifluoromethylsulfonate, trifluoroethylsulfonate or nonafluorobutylsulfonate, particularly the fluoroalkyl sulfonate is trifluoromethylsulfonate.
6. The process for preparing a chemical compound according to any one of items 3 to 5, further comprising a step of removing the protecting group.
7. A process for preparing an alkyl fluoroalkyl sulfonate by reacting an alcohol with a fluoroalkylsulfonic acid anhydride in the presence of a base, wherein the base is of formula NR1R2R3:
   - R1 and R2 are independently 2-methylpropyl or isopropyl; and
   - R3 is -CH(R4)(R5), wherein R4 and R5 are identical or different alkyls that are optionally connected to form a ring.
8. A process for preparing an alkyl fluoroalkyl sulfonate according to item 7, wherein a compound of formula (2) 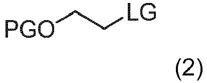 is prepared, the process comprising the step of reacting a compound of formula (3) 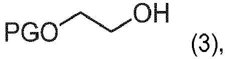 PG being a protecting group; with a fluoroalkylsulfonic acid anhydride, preferably with trifluoromethylsulfonic acid anhydride.
9. The process for preparing a compound of formula (2) according to item 8, wherein the molar ratio of the compound of formula (3) to the base is between 0.5 and 2, preferably is between 0.80 and 1, more preferably is around 0.9.
10. The process for preparing a compound of formula (2) according to item 8 or 9 at a temperature between -10°C and 25°C, preferably around 0°C.
11. The process for preparing a compound of formula (2) according to any one of items 8 to 10, wherein the solvent used for the process is an aprotic organic solvent.
12. The process for preparing a chemical compound comprising reacting rapamycin with an alkyl fluoroalkyl sulfonate in the presence of a base according to any one of items 1 to 6, or the process for preparing an alkyl fluoroalkyl sulfonate according to any one of items 7 to 11, wherein the process is done in a solvent selected from a group consisting of toluene, trifluoromethyltoluene, xylenes, dichloromethane, heptane, pentane, acetonitrile and tert-butylmethyl ether, preferably the solvent is toluene.
13. The process for preparing a chemical compound according to any one of items 1 to 6, wherein the chemical compound is everolimus, the process comprising the steps:
   (a) reacting rapamycin with the compound of formula (2) in the presence of the base 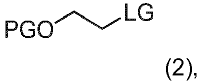 wherein
      - PG is protecting group; and
      - LG is fluoroalkyl sulfonate,
   (b) removing the protecting group to obtain everolimus.
14. The process for preparing a chemical compound according to item 13, wherein the LG is trifluoromethylsulfonate, trifluoroethylsulfonate or nonafluorobutylsulfonate, specifically the LG is trifluoromethylsulfonate.
15. The process for preparing a chemical compound according to item 13, further comprising a step of preparing the compound of formula (2).
16. The process for preparing a chemical compound according to item 14, wherein the compound of formula (2) is prepared according to any one of items 8 to 12.
17. The process for preparing a chemical compound according to any one of items 1 to 7 at a temperature between 25°C and 70°C, preferably between 40°C and 50°C, particularly at 45°C.
18. The process for preparing a chemical compound according to any one of items 1 to 7 and 13 to 15, wherein rapamycin in step (a) is reacted at the temperature between 25°C and 70°C, preferably between 40°C and 50°C, particularly at 45°C.
19. The process for preparing a chemical compound according to any one of items 13 to 18, wherein rapamycin is reacted in an organic aprotic solvent, preferably is selected from the group consisting of toluene, trifluoromethyltoluene, xylenes, dichloromethane, heptane, pentane and mixtures thereof, particularly is toluene.
20. The process for preparing a chemical compound according to any one of items 6 or 13 to 19, wherein the protecting group is removed with an acid.
21. The process of preparing a chemical compound according to any one of items 6 or 13 to 20, wherein the protecting group is removed with HF.pyridine, ammonium fluoride, HF.triethylamine, hexafluoroisopropanol, acetic acid, trifluoroacetic acid, hydrochloric acid, sulfuric acid, or a combination thereof, preferably with HF.pyridine or hexafluoroisopropanol.
22. The process for preparing a chemical compound according to any one of items 6 or 13 to 21, wherein the protecting group is removed in a solvent selected from the group consisting of tetrahydrofuran, methyltetrahydrofuran, acetone, heptane, methanol, acetonitrile and hexafluoroisopropanol, preferably in tetrahydrofuran or hexafluoroisopropanol.
23. The process for preparing a chemical compound according to any one of items 6 or 13 to 22, wherein the protecting group is removed at the temperature between -78°C and 70°C, preferably between 0°C and 70°C.
24. The process for preparing a chemical compound according to any one of items 3 to 23, wherein the protecting group is selected from the group consisting of triisopropylsilyl, tert-butyldimethylsilyl, dimethyltert-hexylsilyl, tert-butyldiphenylsilyl, trityl, benzhydryl, dimethoxyltrityl and diphenylmethyl, preferably is selected from the group consisting of tert-butyldimethylsilyl, tert-butyldiphenylsilyl, trityl and diphenylmethyl, more preferably is tert-butyldimethylsilyl or tert-butyldiphenylsilyl, particularly is tert-butyldiphenylsilyl.
25. The process according to any one of preceding items, wherein a solvent used in the process is free of N,N-dimethylformamide, 1,2-diethoxyethane, 1,2-dimethoxyethane, N,N-dimethylacetamide, Bis(2-methoxyethyl) ether and 1-methyl-2-pyrrolidine.
26. Use of a base having formula NR1R2R3 in preparing everolimus, wherein:
   - R1 and R2 are independently 2-methylpropyl or isopropyl; and
   - R3 is -CH(R4)(R5), wherein R4 and R5 are identical or different alkyls that are optionally connected to form a ring;
   in a chemical reaction comprising an alkyl fluoroalkyl sulfonate.
27. Use of a base having formula NR1R2R3 according to item 26, wherein the fluoroalkylsulfonate part of the alkyl fluoroalkyl sulfonate comprises C1-C4 alkyl substituted by at least one fluoride, preferably the alkyl fluoroalkyl sulfonate being alkyl trifluoromethylsulfonate, alkyl trifluoroethylsulfonate or alkyl nonafluorobutylsulfonate, specifically the alkyl fluoroalkyl sulfonate is alkyl trifluoromethylsulfonate.
28. Use of a base having formula NR1R2R3 according to item 28 or 29, the alkyl part of the alkyl fluoroalkyl sulfonate is substituted with a functional group, wherein the functional group is protected with a protecting group.
29. Use of a base having formula NR1R2R3 according to items 28 or 29, wherein the functional group is -OH, -SH or -NH2 group, preferably is -OH.
30. Use of a base having formula NR1R2R3 according to any one of items 26 to 29, wherein the alkyl fluoroalkyl sulfonate is alkyl trifluoromethanesulfonate.
31. Use of a base having formula NR1R2R3 according to item 26 to 30, wherein the alkyl fluoroalkyl sulfonate is alkyl fluoroalkyl sulfonate of formula (2): 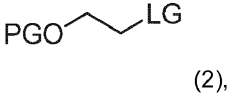 wherein
   - PG is protecting group; and
   - LG is fluoroalkyl sulfonate.
32. Use of a base having formula NR1R2R3 according to item 31, wherein the fluoroalkylsulphonsulfonate is trifluoromethylsulfonate, trifluoroethylsulfonate or nonafluorobutylsulfonate, specifically the fluoroalkyl sulfonate is trifluoromethylsulfonate.
33. The process according to any one of items 1 to 25, or use according to any one of items 26 to 32, wherein the base has formula (4) 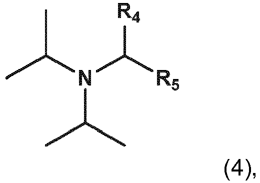 wherein R4 and R5 are identical or different alkyls that are optionally connected to form a ring;
   or formula (5) 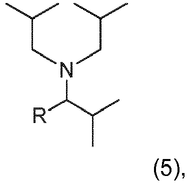 wherein R is an alkyl.
34. The process according to item 33, or use according to item 33, wherein R4 and R5 are both ethyl or both butyl; or R is isopropyl.
35. The process according to item 33 or 34, or use according to item 33 or 34, wherein R4, R5 are propyl.
36. The process according to any one of items 1 to 25, or use according to any one of items 26 to 32, wherein the base is N,N-diisopropylpentan-3-amine.
37. The process for preparing a chemical compound according to any one of items 3 to 12 or 13 to 25, or use according to any one of items 26 to 32, wherein the base is N,N-diisopropylpentan-3-amine and the protecting group is selected from a group consisting of tert-butyldiphenylsilyl, dimethyl tert-hexylsilyl, tert-butyldimethylsilyl and trityl, particularly is tert-butyldiphenylsilyl.
38. The process for preparing a chemical compound according to any one of items 3 to 12 or 13 to 25, or use according to any one of items 26 to 32, wherein the base is N,N-diisopropylpentan-3-amine and the protecting group is trityl.
39. The process for preparing a chemical compound according to any one of items 12 to 25, or 34 to 37, wherein the molar ratio of the alkyl trifluoromethanesulfonate to rapamycin is between 4 and 1.5, preferably is between 2 and 3, more preferably is 2.5.
40. The process for preparing a chemical compound according to any one of items 3 to 12 or 13 to 25, or use according to any one of items 26 to 32, wherein the base is N,N-diisopropylnonan-5-amine and the protecting group is selected from a group consisting of tert-butyldiphenylsilyl, dimethyl tert-hexylsilyl, tert-butyldimethylsilyl and trityl, particularly is tert-butyldiphenylsilyl.
41. The process for preparing a chemical compound according to any one of items 3 to 12 or 13 to 25, or use according to any one of items 26 to 32, wherein the base is N,N-diisopropylnonan-5-amine and the protecting group is trityl.
42. The process for preparing a chemical compound according to any one of items 3 to 12 or 13 to 25, or use according to any one of items 25 to 32, wherein the base is N,N-diisobutyl-2,4-dimethylpentan-3-amine and the protecting group is selected from a group consisting of tert-butyldiphenylsilyl, dimethyl tert-hexylsilyl, tert-butyldimethylsilyl and trityl, particularly is tert-butyldiphenylsilyl.
43. The process for preparing a chemical compound according to any one of items 3 to 12 or 13 to 25, or use according to any one of items 26 to 31, wherein the base is N,N-diisobutyl-2,4-dimethylpentan-3-amine and the protecting group is trityl.
44. The process according to any one of items 1 to 25, or use according to any one of items 26 to 32, wherein the base is N,N-diisopropylnonan-5-amine.
45. The process according to any one of items 1 to 25, or use according to any one of items 26 to 32, wherein the base is N,N-diisobutyl-2,4-dimethylpentan-3-amine.
46. A process for preparing a pharmaceutical formulation, the process comprising the steps for preparing a chemical compound according to any one of items 1 to 7, 12 to 25, or 33 to 45, and mixing the chemical compound with at least one pharmaceutically acceptable excipient.49. The process according to item 46, wherein the chemical compound is everolimus.

### Detailed description of the Invention

Surprisingly we found a process for preparing a chemical compound comprising reacting rapamycin with an alkyl fluoroalkyl sulfonate in the presence of a base, wherein the base is of formula NR1R2R3:
- R1 and R2 are independently 2-methylpropyl or isopropyl; and
- R3 is -CH(R4)(R5), wherein R4 and R5 are identical or different alkyls that are optionally connected to form a ring. The same base can be used in the process of preparing an alkyl fluoroalkyl sulfonate as a final chemical compound or a reagent for later use. To prepare the alkyl fluoroalkyl sulfonate the corresponding alcohol is reacted with a fluoroalkylsulfonic acid anhydride in the presence of the base. As a matter of fact, the base would be beneficial when used as a base in any chemical reaction comprising an alkyl fluoroalkyl sulfonate as a reagent since it would improve the efficiency of the reaction by preventing side-reactions from occurring.

The bases defined herein are unusual bases, but they surprisingly improve the stability of the alkyl fluoroalkyl sulfonate in the reaction mixture as compared to other bases. So far bases like 2,6-lutidine, pyridine, triethylamine, diisopropylamine or N,N-diisopropylethylamine have been disclosed for similar alkylation procedures in the chemical literature. However, the said bases can cause alkyl fluoroalkyl sulfonate to decompose during the reaction, which leads to formation of unnecessary side products. On the other hand, use of the bases shown herein causes the chemical synthesis to run with less side reactions and thus less side products are formed. The new process comprising the aforementioned base results in higher yields and less impurities, which makes a later work-up to isolate and purify the product of a chemical reaction easier and again more efficient.

The substituents of the base R4, R5 and R according to the present disclosure are alkyls or R4 and R5 together form a cyclo alkyl. The term "alkyl" as used herein denotes a straight or branched (singly, if desired and possible, more times) carbon chain of C2-C10-alkyl, such as C2-C5-alkyl, in particular branched C2-C5-alkyl, such as isopropyl or linear C2-C5-alkyl, such as ethyl. The term "C2-C10-" defines a moiety with up to and including maximally 10, especially up to and including maximally 5, carbon atoms, said moiety being branched (one or more times) or straight-chained and bound via a terminal or a non-terminal carbon. C2-C10-alkyl, for example, is n-pentyl, n-hexyl or n-heptyl or preferably C2-C5-alkyl, especially, ethyl, n-propyl, iso-propyl, n-butyl, isobutyl, sec-butyl, tert-butyl, n-hexyl and, in particular ethyl, n-propyl, iso-propyl, n-butyl, isobutyl, sec-butyl, tert-butyl; preferably ethyl, iso-propyl or n-butyl. In one embodiment R4 and R5 are both ethyl. In another embodiment R4 and R5 are both butyl. "Cyclo alkyl" denotes when, alternatively, the R4 and R5 alkyls form a ring of for example C5 or C6 carbon atoms. One example of possible cycloalkyl is cyclohexyl. Specifically, the bases that have shown the best results and are thus preferred according to the present invention are depicted just below: 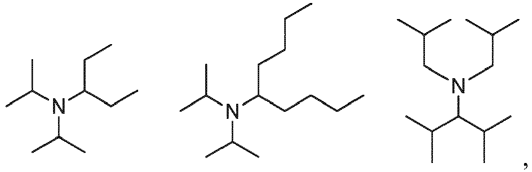 i.e. the bases are N,N-diisopropylpentan-3-amine, N,N-diisopropylnonan-5-amine and N,N-diisobutyl-2,4-dimethylpentan-3-amine, respectively. In the present disclosure isobutyl and 2-methylpropyl are interchangeable. The bases described herein can be prepared for example according to the process described in Liebigs Ann. Chem. 1985, 2178-2193 or Liebigs Ann. Chem. 1974, 1543 - 1549.

According to the present invention, the bases are used in a process, where rapamycin is reacted with an alkyl fluoroalkyl sulfonate in the presence of a base. Particularly, the process of the present disclosure can be applied when rapamycin (sirolimus) is used as a starting material.

"Fluoroalkyl sulfonate" means herein a sulfonate group of formula -O-S(O)₂-fluoroalkyl comprising at least a mono fluorinated C1-C4 alkyl component. It is apparent that the more fluorinated the C1-C4 fluoroalkyl component is, the better leaving group it forms and thus more susceptible it is to degradation by an improperly selected base. The best leaving groups are perfluoroalkyl sulfonates. In a preferred embodiment, trifluoromethylsulfonate, nonafluorobutylsulfonate or trifluoroethylsulfonate is used. The molar ratio of the alkyl fluoroalkyl sulfonate to the nucleophile in the process of the present disclosure is typically between 4 and 1.5, preferably is between 2 and 3, and more preferably is about 2.5.

The alkyl part of an alcohol that is esterified with the fluoroalkyl sulfonic acid to form the alkyl fluoroalkyl sulfonate is described by the term "alkyl" as defined above. The alkyl part (i.e. alcohol part) of the alkyl fluoroalkyl sulfonate can be further substituted. In one embodiment the alkyl moiety is substituted with a functional group, which can be for example -OH, -SH or -NH2 group. In case the functional group is -OH or -NH2 or any other reactive group, it is best to have it protected by a protecting group, otherwise it could cause further side reactions. In one embodiment the alkyl part of the alkyl fluoroalkyl sulfonate is ethane substituted with -OH. In the main embodiment, the -OH group is protected by a protecting group. In a preferred embodiment, the alkyl fluoroalkyl sulfonate as used herein is alkyl trifluoromethylsulfonate, alkyl nonafluorobutylsulfonate or alkyl trifluoroethylsulfonate, particularly is alkyl trifluoromethylsulfonate. When the alkyl part is PG-O-ethyl-, the alkyl fluoroalkyl sulfonate forms the compound of formula (2). Particularly the reagents PG-O-ethyl-trifluoromethylsulfonate, PG-O-ethyl-nonafluorobutylsulfonate or PG-O-ethyl-trifluoroethylsulfonate can be used in the process of the present disclosure; specifically the reagent is PG-O-ethyl-trifluoromethylsulfonate. The protecting group can be for example triisopropylsilyl, tert-butyldimethylsilyl, dimethyltert-hexylsilyl, tert-butyldiphenylsilyl, trityl, benzhydryl, dimethoxyltrityl. Protecting groups can optionally be introduced according to the following references: tert-butyldimethylsilyl according to WO2007/124898 A1, 2007 or Org. Lett., 2006, 8, 5983 - 5986; tert-butyldiphenylsilyl according to Tetrahedron Lett., 2000, 41, 4197 - 4200; triisopropylsilyl according to J. Med. Chem. 2006, 49, 2333 -2338; dimethyltert-hexylsilyl according to WO2012/103959 A1, 2012; benzhydryl according to Org. Biomol. Chem., 2012, 10, 1300; and trytil according to J. Org. Chem. 1992, 57, 6678 - 6680. In any event in the reactions mentioned hereinbefore and hereinafter, protecting groups may be used where appropriate or desired, even if this is not mentioned specifically, to protect functional groups that are not intended to take part in a given reaction, and they can be introduced and/or removed at appropriate or desired stages. Reactions comprising the use of protecting groups are therefore included as possible wherever reactions without specific mentioning of protection and/or deprotection are described in this specification. Within the scope of this disclosure only a readily removable group that is not a constituent of the particular desired end product is designated a "protecting group", unless the context indicates otherwise. The protection of functional groups by such protecting groups, the protecting groups themselves, and the reactions appropriate for their introduction and removal are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974.

Because the alkyl fluoroalkyl sulfonate is sensitive to decomposition, and potentially the nucleophilic starting material is also unstable under the reaction conditions employed, the solvents selected for the process is advantageously a solvent which is unreactive towards the alkyl fluoroalkyl sulfonate or the nucleophile used and which is itself unreactive under the reaction conditions employed. Generally the solvent may be an inert solvent like organic aprotic solvent, preferably nonpolar aprotic solvent. Examples of such unreactive solvents include but are not limited to those selected from the group consisting of aliphatic, cyclic or aromatic hydrocarbons, including but not limited to those selected from the group consisting of hexane, pentane, heptane, toluene, cyclohexane, octane, mixtures thereof, etc.; chlorinated hydrocarbons, including but not limited to those selected from the group consisting of dichloromethane, aliphatic (linear or branched) or cyclic ether of 4 to 10 carbon atoms and 1 to 3 oxygen atoms, and alkylnitril, and mixtures thereof. Preferably the solvent is toluene, trifluoromethyltoluene, xylenes, dichloromethane, heptane, pentane, acetonitrile, or tert-butylmethyl ether, or mixtures thereof. More preferably the solvent is toluene. Solvents can optionally be completely devoid of water, thus they can be stored or treated by dessicants. Additionally, the solvent can be deaerated by an inert gas like for example nitrogen or argon. The temperature of the reaction can be adjusted according to the reagents charged in the reaction mixture, but will preferably be in the range from -80 °C to 90 °C, more preferably from -50 °C to 70 °C. Depending on the best reaction conditions the reaction temperature can be set from -10 °C to 25 °C, or from 25°C to 50°C.

Preferably the solvent used in the reaction does not comprise N,N-dimethylformamide, 1,2-diethoxyethane, 1,2-dimethoxyethane, N,N-dimethylacetamide, Bis(2-methoxyethyl) ether, 1-methyl-2-pyrrolidinedimethoxyethane or similar polar aprotic solvent. Said polar aprotic solvents are commonly used in nucleophilic substitution reactions to enhance reactivity. However, in a process of a present invention said solvents can be avoided. Polar aprotic solvents accelerate side reactions and thus decomposition of alkyl fluoroalkyl sulfonate reactants. In addition, using solvents devoid of polar aprotic solvents listed above serves the benefit of human health and the environment.

### Preferred embodiments of the present invention

The preferred embodiments of the present invention relate to the processes of the present invention for the preparation of active pharmaceutical ingredient (API), particularly everolimus. It was shown that by replacing the bases that have been normally used in chemical reactions involving alkyl fluoroalkyl sulfonates with the bases as specified herein, the API such as everolimus can be prepared in higher yields with less impurities in a more efficient manner. These aspects are especially important in the field of pharmaceuticals, where impurity profile of the API is of a particular importance. In addition, better process efficiency facilitates process scale-up, which can in turn lead to production volumes that can satisfy the demand.

A process for preparing everolimus can first be led to the protected intermediate, wherein rapamycin is reacted with an alkyl fluoroalkyl sulfonate in the presence of a base, wherein the base is of formula NR1R2R3:
- R1 and R2 are independently 2-methylpropyl or isopropyl; and
- R3 is -CH(R4)(R5), wherein R4 and R5 are identical or different alkyls that are optionally connected to form a ring. All specifics of the second step described below apply.

Generally, the process for preparing everolimus can comprise the steps:
(a) reacting rapamycin with the compound of formula (2) (a second step as described below). 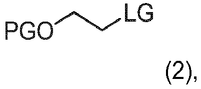 wherein
   - PG is protecting group; and
   - LG is fluoroalkyl sulfonate,
   in the presence of the base of formula NR1R2R3:
   - R1 and R2 are independently 2-methylpropyl or isopropyl; and
   - R3 is -CH(R4)(R5), wherein R4 and R5 are identical or different alkyls that are optionally connected to form a ring,
(b) removing the protecting group to obtain everolimus. To this process, a first step of preparing the compound of formula (2) can be added.

In a first step, a reactant, i.e. an alkyl fluoroalkyl sulfonate, can be prepared by a process of reacting an alcohol with a fluoroalkylsulfonyic acid anhydride in the presence of the base as defined above. Generally, even other bases such as for example N,N-diisopropylethylamine can be used in this step. Such process allows the preparation of a compound of formula (2) 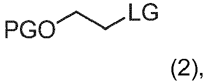 wherein LG denotes fluoroalkyl sulfonate, i.e. the leaving group of formula -O-S(O)₂-fluoroalkyl, and PG is protecting group. Preferably the LG is trifluoromethylsulfonate, nonafluorobutylsulfonate or trifluoroethylsulfonate. In a particular embodiment the LG denotes trifluoromethylsulfonate. The reaction can be executed by a process comprising the step of reacting a compound of formula (3)

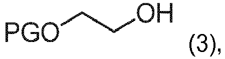

PG being a protecting group; with a fluoroalkylsulfonic acid anhydride in a following manner:

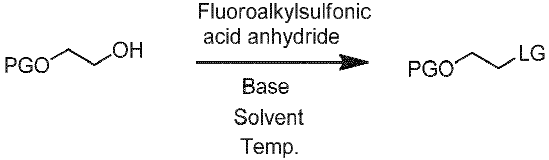

In a preferred embodiment the fluoroalkylsulfonic acid anhydride is trifluoromethylsulfonic acid anhydride, nonafluorobutylsulfonic acid anhydride or trifluoroethylsulfonic acid anhydride. Specifically the fluoroalkylsulfonic acid anhydride is trifluoromethylsulfonic acid anhydride. By choosing a fluoroalkylsulfonic acid anhydride the corresponding fluoroalkyl sulfonate leaving group (LG) represented by -O-S(O)₂-fluoroalkyl is obtained on the compound of formula (2). The base can be any suitable base of weak nucleophilicity, but in order to secure the stability of the product, enhance the reaction yield, reduce by-product formation, and make purification more straightforward, preferably the base of formula NR1R2R3 is used:
- R1 and R2 are independently 2-methylpropyl or isopropyl; and
- R3 is -CH(R4)(R5), wherein R4 and R5 are identical or different alkyls that are optionally connected to form a ring. Particularly preferred bases are N,N-diisopropylpentan-3-amine, N,N-diisopropylnonan-5-amine or N,N-diisobutyl-2,4-dimethylpentan-3-amine. The molar ratio of the compound of formula (3) to the base is between 0.5 and 2, preferably is between 0.80 and 1, more preferably is around 0.9. The reaction temperature in the process is adjusted to between -10°C and 25°C, preferably around 0°C. The solvent used is the solvent as described above, i.e. an inert solvent like organic aprotic solvent, preferably nonpolar aprotic solvent. Preferably the solvent is selected from a group consisting of toluene, trifluoromethyltoluene, xylenes, dichloromethane, heptane, pentane, acetonitrile and tert-butylmethyl ether, and mixtures thereof. Most preferably the solvent is toluene.

The protecting group used in the process of preparing the compound of formula (2) is as described above any protecting group that a skilled person would select based on general textbook references. The following protecting groups are especially preferred: triisopropylsilyl, tert-butyldimethylsilyl, dimethyltert-hexylsilyl, tert-butyldiphenylsilyl, trityl, benzhydryl, dimethoxyltrityl and diphenylmethyl, preferably the protecting group is selected from the group consisting of tert-butyldimethylsilyl, tert-butyldiphenylsilyl, trityl, dimethoxytrityl and diphenylmethyl, more preferably is tert-butyldimethylsilyl or tert-butyldiphenylsilyl, particularly is tert-butyldiphenylsilyl. As a general guidance for preparing the compound of formula (2), the procedure can resemble the following one:
- To a solution of the protected ethylene glycol derivative (1 equiv.) in solvent (0.57 M) was added the amine base (1.05-1.14 equiv.). The solution was then cooled to 0 °C and trifluoromethanesulfonic acid anhydride (0.97-1.0 equiv.) was added dropwise such that the temperature was maintained between (-2°C - 2°C). The reaction was allowed to warm to ambient temperature and stirred for a further 1 h. GC-MS analysis indicated that the triflate formation was complete after this time.

The process for preparing the compound of formula (2) runs best in the combination of the protecting group being tert-butyldimethylsilyl, solvent being toluene and the base being N,N-diisopropylethylamine; or protecting group being tert-butyldiphenylsilyl, solvent being toluene and the base being N,N-diisopropyl-pentan-3-amine.

In a second step, a nucleophile rapamycin is alkylated with the compound of formula (2) in the presence of a base, where the base is of formula NR1R2R3:
- R1 and R2 are independently 2-methylpropyl or isopropyl; and
- R3 is -CH(R4)(R5), wherein R4 and R5 are identical or different alkyls that are optionally connected to form a ring.

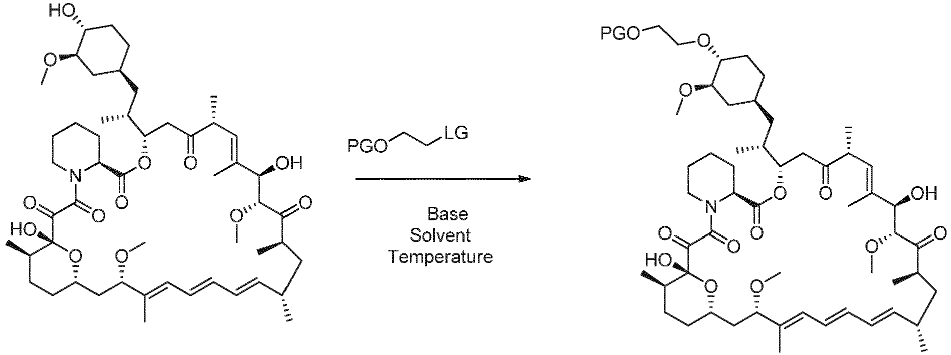

Particularly preferred bases are N,N-diisopropylpentan-3-amine, N,N-diisopropylnonan-5-amine or N,N-diisobutyl-2,4-dimethylpentan-3-amine. The process of reacting rapamycin with the compound of formula (2) is best carried out at a temperature between 25°C and 70°C, preferably between 40°C and 50°C, particularly at 40°C in an organic aprotic solvent as described above. Particularly well does the process run in toluene, trifluoromethyltoluene, xylenes, dichloromethane, heptane, pentane, or mixtures thereof. The most preferred solvent for the reaction is toluene. Protecting group suitable for the process include but are not limited to triisopropylsilyl, tert-butyldimethylsilyl, tert-hexyldimethylsilyl, tert-butyldiphenylsilyl, trityl, dimethoxyltrityl, and benzhydryl. The reaction can for example resemble the process:
- To a solution of the triflate as prepared in the first step above (1.5 to 5 equiv.) a base was added (1.73 to 5.75 equiv.) followed by rapamycin (1 equiv.). Further solvent was used for washing to provide a final concentration of 0.11 M with respect to rapamycin. The reaction was heated to the appropriate temperature and monitored by HPLC.

The best combination of reaction conditions for this step is the combination of N,N-diisopropylpentan-3-amine base, tert-butyldiphenylsilyl protecting group and the solvent being toluene. The best temperature to select with the above specific conditions for the second step is between about 40°C and 50°C.

The protected intermediate in a reaction mixture can be purified by silica gel chromatography. Particularly, the technique is suitable to purify crude reaction mixture of silylprotected everolimus. Solvents used for purification can be a mixture of an ester, such as ethyl acetate or isopropyl acetate, and non-polar aliphatic solvent such as n-heptane, n-hexane, heptane isomer, hexane isomer, or mixtures thereof. These solvent systems ensure sufficient solubility of a crude product, its separation and elution. Systems based on toluene and methylisobutylketone, methyl ethyl ketone, isopropanol and ethanol fail due to suboptimal solvent strength, solubility of a co-solvent, or solubility of the crude product in these solvents. Addition of a polar and protic co-solvent, such as water or methanol, in up to 5 volume % (e.g. from 1-5 volume %) increases stability of a target molecule on stationary phase by weakening acidity of silanol groups. In addition, the reaction mixture can be pretreated by precipitation or crystallization of salt compounds formed as by-products. Subsequently the by-products can be adsorbed on silica gel and removed by filtration, which can be followed by a subsequent purification based on normal phase silica gel chromatography. The selective precipitation (crystallization) and adsorption process can improve long term stability of a stationary phase and avoid the need for tedious cleaning-in-place between runs or additional pre-treatment of a reaction mixture with disposable filter cartridges for removal of salt compounds. Improved purity is achieved by a choice of a stationary phase that displays acceptable selectivity. Overall, the purification between the reaction steps allows efficient removal of a specific by-product that stems from a chemical conversion of an impurity of the starting material. Purity of a final chemical compound depends also on the efficiency of the removal of said by-product already at this stage.

This purification step enables to control precursor of critical impurity ethyl-everolimus to levels of about 0.2%. At the same time, it ensures stability of the target product throughout the purification process. In addition, it is a robust process and can be scaled up to full commercial batches in industrial settings.

The next step in preparing everolimus is the cleavage of a protecting group. Therefore, the process of preparing everolimus can comprise steps:
(a) reacting rapamycin with the compound of formula (2) in the presence of the base,
(b) removing the protecting group to obtain everolimus.

The process of preparing everolimus can extend further to formulating a pharmaceutical composition comprising the obtained everolimus.

Removal of the protecting group can be carried out under standard reaction conditions known in the art, unless otherwise specified, preferably those mentioned specifically, in the absence or, customarily, in the presence of acids or bases, preferably acids or bases that cause removal of the protecting group but at the same time do not cause chemical degradation of everolimus. Preferably, the protecting group is removed with an acid. Particularly suitable acids for the removal of the protecting group from everolimus are HF.pyridine, HF.triethylamine ammonium fluoride, hexafluoroisopropanol, acetic acid, trifluoroacetic acid, hydrochloric acid, sulfuric acid, or a combination thereof, preferably the acid is HF.pyridine or hexafluoroisopropanol. The removal of the protecting group can take place in the same solvent as was used in the previous reaction steps. However, the solvent can be replaced with one that facilitates the removal of the protecting group. As an example, the protecting group can be removed in a solvent selected from the group consisting of tetrahydrofuran, methyltetrahydrofuran, acetone, heptane, methanol, acetonitrile and hexafluoroisopropanol, preferably in tetrahydrofuran or hexafluoroisopropanol. The protecting group is removed at the temperature between -78°C and 70°C, preferably between 0°C and 70°C. The deprotection reaction is run best with HF.Pyridine in tetrahydrofuran, optionally at ambient temperature.

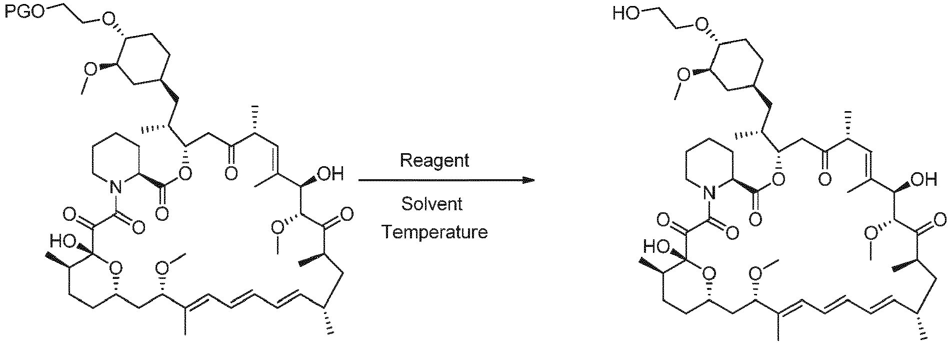

Surprisingly it has been found that specific protecting group can amplify the overall reaction yields, when used in the combination with the specific bases as described herein. Such protecting group can be selected from a group consisting of triisopropylsilyl, tert-butyldimethylsilyl, dimethyltert-hexylsilyl, tert-butyldiphenylsilyl, trityl, benzhydryl, dimethoxyltrityl or diphenylmethyl, preferably is selected from the group consisting of tert-butyldimethylsilyl, tert-butyldiphenylsilyl, trityl and diphenylmethyl, more preferably tert-butyldimethylsilyl and tert-butyldiphenylsilyl, and particularly is tert-butyldiphenylsilyl. Selection of the base described in the present invention for the preparation of everolimus in a specific combination with the triisopropylsilyl, tert-butyldimethylsilyl, dimethyltert-hexylsilyl, tert-butyldiphenylsilyl, trityl, benzhydryl, or diphenylmethyl protecting group, preferably tert-butyldimethylsilyl or tert-butyldiphenylsilyl, particularly tert-butyldiphenylsilyl protecting group, allow the process for preparing everolimus to be conducted with advantageously high yields, especially when combined with N,N-diisopropylpentan-3-amine, N,N-diisopropylnonan-5-amine or N,N-diisobutyl-2,4-dimethylpentan-3-amine base, particularly N,N-diisopropylpentan-3-amine.

In particular embodiment N,N-diisopropylpentan-3-amine and the protecting group tert-butyldiphenylsilyl are selected for the process of preparing everolimus according to the present disclosure.
In another particular embodiment N,N-diisopropylpentan-3-amine and protecting group tert-butyldimethylsilyl are selected for the process of preparing everolimus.
In yet another embodiment N,N-diisopropylpentan-3-amine and protecting group trityl are selected for the process of preparing everolimus.
In a further embodiment, the base N,N-diisopropylnonan-5-amine is used in the process of the present disclosure in combination with tert-butyldiphenylsilyl protecting group. N,N-diisopropylnonan-5-amine can also be used in combination with trityl protecting group.
In addition, also N,N-diisobutyl-2,4-dimethylpentan-3-amine can be used in the process of the present disclosure in combination with the tert-butyldiphenylsilyl protecting group. A combination of N,N-diisobutyl-2,4-dimethylpentan-3-amine with the trityl protecting group is also specifically envisaged in the present disclosure. N,N-diisobutyl-2,4-dimethylpentan-3-amine and N,N-diisopropylnonan-5-amine can also be used together with tert-butyldimethylsilyl protecting group.

After deprotection reaction is completed the workup process and purification are selected as appropriate according to the physicochemical properties of the chemical compound obtained. In case of compounds such as Everolimus, the reaction mixture is neutralised and the product (such as everolimus) extracted by a water-immiscible organic solvent and isolated from the organic phase. After isolation the product (i.e. everolimus) can be further washed, dried and purified by methods known to a skilled person. By using the bases as defined in claim 1, optionally in combination with the preferred protecting group, in the process of the present disclosure, the purity of the synthesized product is higher. This makes the purification simpler with less purification steps required. The obtained product can be used for further synthesis or as an end product. Excipients like for example colorants or antioxidants can be added. In the event that the product is API, like in the case of everolimus, the compound can be further stabilized with an antioxidant (like for example 2,6-di-tert-butyl-4-methylphenol, known also as butylhydroxytoluol or BHT) and packed in bulk and/or formulated in a pharmaceutical composition. Formulating a pharmaceutical formulation in general means mixing the obtained API such as everolimus with at least one pharmaceutically acceptable excipient, e.g. appropriate carrier and/or diluent. Excipients include, but are not limited to fillers, binders, disintegrants, flow conditioners, lubricants, sugars or sweeteners, fragrances, preservatives, stabilizers, wetting agents and/or emulsifiers, solubilizers, salts for regulating osmotic pressure and/or buffers. Depending on the form of a pharmaceutical composition and the route of administration a skilled person would select proper excipients. Form of a pharmaceutical formulation can be for example coated or uncoated tablet, capsule, (injectable) solution, infusion solution, solid solution, suspension, dispersion, solid dispersions, cream, gel, ointment, paste, inhaler powder, foam, tincture, suppository or stent (or layer of a stent). Equally, a skilled person would know how to select a suitable route of administration: for example would administer API or pharmaceutical formulation enterally or parenterally, or via medical device, e.g. for local delivery like in the case of a stent.

The following Examples serve to illustrate the invention without limiting the scope thereof, while they on the other hand represent preferred embodiments of the reaction steps, intermediates and/or the process of manufacture of the product in free base form or as a pharmaceutically acceptable salt thereof.

### Example 1 (TBS or tert-butyldimethylsilyl protecting group)

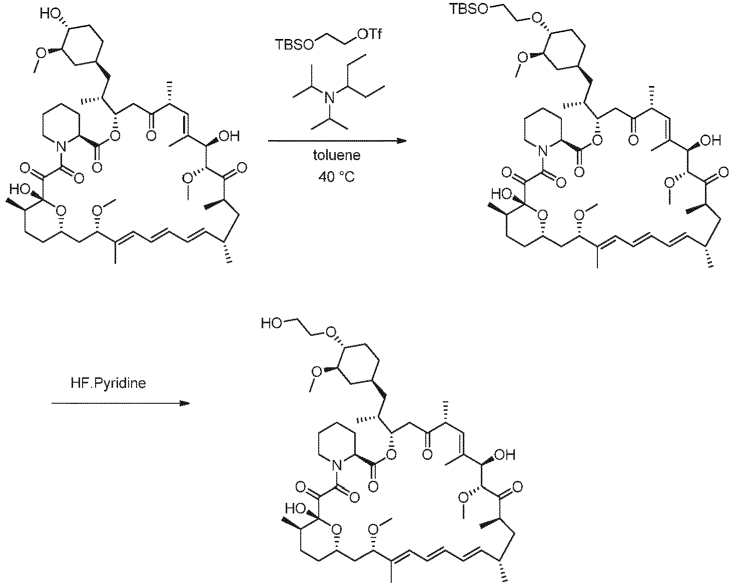

To a solution of 2-((tert-butyldimethylsilyl)oxy)ethanol (8.04 g, 43.8 mmol) in toluene (55 g) was added N,N-diisopropylethylamine (5.94 g, 45.9 mmol). The clear solution was then cooled to 0°C and trifluoromethanesulfonic acid anhydride (11.97 g, 42.4 mmol) was added dropwise such that the temperature was maintained between (-2°C - 2°C). Following the addition a further portion of toluene (5 g) was used for washing. After 30 minutes N,N-diisopropylpentan-3-amine (7.871 g, 45.9 mmol) was added followed by toluene (3 g) and Rapamycin (10.0 g, 10.9 mmol) washing with toluene (18.4 g). The reaction was then heated to 40 °C and allowed to stir at this temperature for 42 h at which point less than 5 Area% Rapamycin was remaining according to HPLC analysis. The reaction was cooled to ambient temperature and pyridine (2.6 g) was then added to quench the reaction which was stirred for a further 30 minutes. The reaction was filtered and diluted with isopropyl acetate. The organic solution was washed with 1M citric acid solution, 10% sodium bicarbonate solution followed by water, dried (MgSO₄) and concentrated in vacuo. The residue was divided into two portions.

To half of this crude residue (9.66 g) THF (100 mL) was added and this solution was then added dropwise at 0 °C to a HF*pyridine solution (1:1, 17.7 g). A further portion of THF (20 mL) was used for washing. The reaction was heated to 45 °C for 1.5 h, then allowed to cool to ambient temperature and diluted with isopropylacetate (150 g). The reaction was then added slowly to an 8% aqueous solution of sodium bicarbonate and further washed with isopropylacetate (250 g). The organic phase was then separated and washed with saturated aqueous sodium chloride solution, dried (MgSO₄) and concentrated in vacuo.

The residue was diluted with isopropylacetate, butylhydroxytoluol (BHT; 0.2% m/m) was added, and the yield of everolimus determined by HPLC analysis against an external standard (2.96 g, 57%).

### Example 2 (TBDPS or tert-butyldiphenylsilyl protecting group).

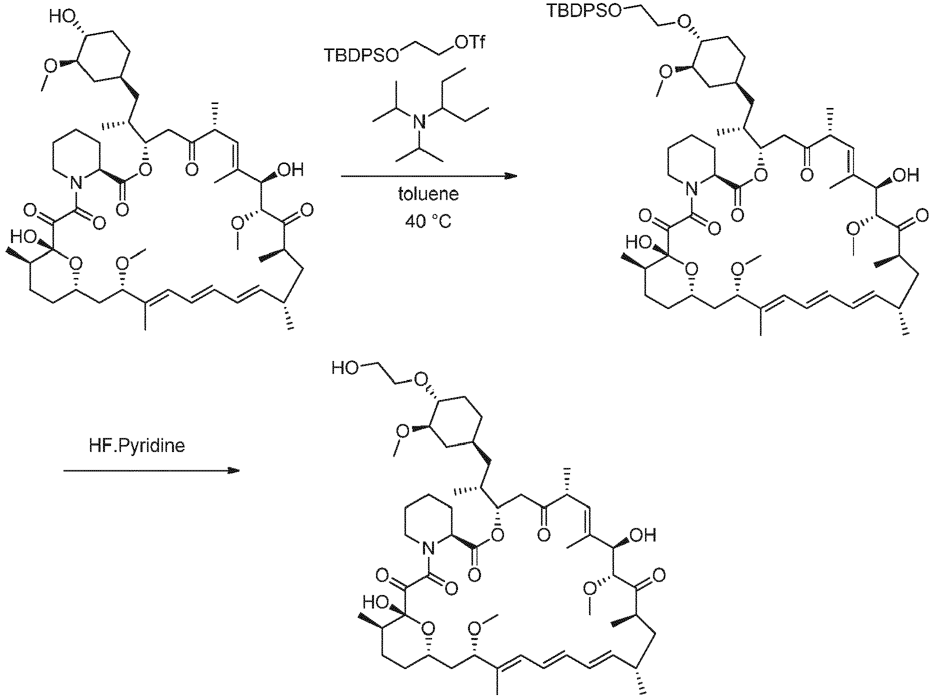

To a solution of 2-((tert-butyldiphenylsilyl)oxy)ethanol (13.1 g, 43.8 mmol) in toluene (51 g) was added N,N-diisopropylpentan-3-amine (8.7 g, 50.3 mmol). The clear solution was then cooled to 0°C and trifluoromethanesulfonic acid anhydride (12.3 g, 43.8 mmol) was added dropwise such that the temperature was maintained between (-2°C - 2°C). Following the addition a further portion of toluene (5 g) was used for washing.

After 1.5 h N,N-diisopropylpentan-3-amine (8.7 g, 50.3 mmol) was added followed by toluene (3 g) and Rapamycin (10.0 g, 10.9 mmol) washing with toluene (18 g). The reaction was then heated to 40°C and allowed to stir at this temperature for 22.5 h at which point less than 5 Area% Rapamycin was remaining according to HPLC analysis. The reaction was cooled to ambient temperature and pyridine (1.0 mL) was then added to quench the reaction which was stirred for a further 30 minutes. The reaction was filtered and diluted with isopropyl acetate. The organic solution was washed with 1M citric acid solution, 10% sodium bicarbonate solution followed by water, dried (MgSO₄) and concentrated in vacuo. To the crude residue (35.6 g) was added THF (240 mL) and this solution was then added dropwise at 0 °C to a HF*pyridine solution (1:1, 38.1 g). The reaction was heated to 45 °C for 3.5 h then allowed to cool to ambient temperature and diluted with isopropylacetate (300 g). The reaction was then added slowly to an 8% aqueous solution of sodium bicarbonate and further washed with isopropylacetate (250 g). The organic phase was then separated and washed with saturated aqueous sodium chloride solution, dried (MgSO₄) and concentrated in vacuo. The residue was diluted with isopropylacetate, BHT (0.2% m/m) was added, and the yield of everolimus determined by HPLC analysis against an external standard (6.81 g, 65%).

### Example 3 (Dimethoxytrityl protecting group)

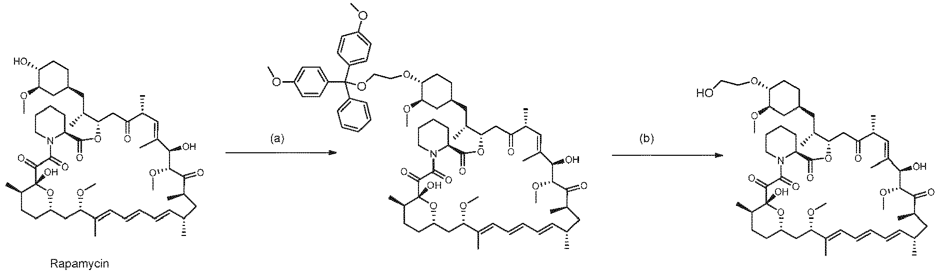

### Step (a)

2-(bis(4-methoxyphenyl)(phenyl)methoxy)ethanol (3.049 g, 8.366 mmol) was dissolved in toluene (10.2g) and then N,N-diisopropylpentan-3-amine (1.648 g, 9.621 mmol) followed by toluene (0.5 g) were added. The reaction mixture was cooled to 0°C and trifluoromethanesulfonic acid anhydride (1.376 ml, 8.145 mmol) was added dropwise followed by toluene (1 g). The reaction mixture was allowed to warm to room temperature and stirred for a further 1 h at this temperature. A further portion of N,N-diisopropylpentan-3-amine (1.648 g, 9.621 mmol) was then added followed by toluene (1 g). Rapamycin (2 g, 2.092 mmol) was then added in a single portion and washed in with toluene (3.7 g). The reaction was then heated to 40 °C for 18 h before cooling to ambient temperature and addition of pyridine (0.20 g). The reaction was then stirred for 30 minutes and diluted with isopropylacetate (50 mL). This organic phase was then washed with 1M citric acid (2 x 40 mL) with aqueous sodium bicarbonate solution (8%, 26 g) and finally with water (2 x 20 mL) before drying (MgSO₄) and concentration in vacuo. The crude product was dissolved in the minimal amount of dichloromethane and purified via flash column chromatography (15-100% ethyl acetate in heptanes) to afford the desired product as a yellow oil.

### Step (b)

This intermediate was then dissolved in acetone (8.2 mL) and heptane (9 mL) was added followed by water (4.6 g). The biphasic mixture was then cooled to 10°C and acetic acid (3.45 mL) was added dropwise. The reaction was stirred at this temperature for 18 h then diluted with isopropylacetate (15 g) and cooled to 0°C. An aqueous solution of sodium hydroxide (15%, 17 g) was then added dropwise and the phases were separated. The aqueous phase was extracted with isopropylacetate (2 x 7.2 g) and the combined organic fractions were washed with water, dried (MgSO₄) and concentrated in vacuo to yield colorless oil.

HPLC analysis of the product everolimus against an external standard showed a yield of 381 mg, 19% over the two steps.

### Example 4 (Dimethoxytritylprotecting group)

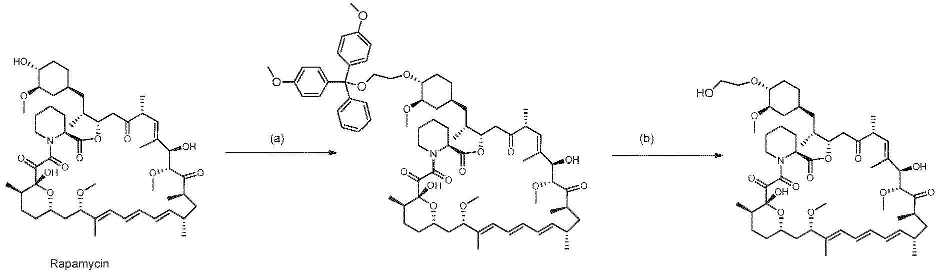

Step (a) was performed as above and then the resulting intermediate after chromatography was dissolved in hexafluoroisopropanol (26 mL). The reaction was heated for 1.5 h at 50 °C and then cooled to 0 °C and quenched with saturated aqueous sodium bicarbonate solution (26 mL). The organic phase was separated and washed with saturated aqueous sodium chloride solution, water, dried (MgSO₄) and concentrated. HPLC analysis of the product everolimus against an external standard showed a yield of 621 mg, 31% over the two steps.

### Example 5 (Tert-butyl-diphenylsilyl protecting group)

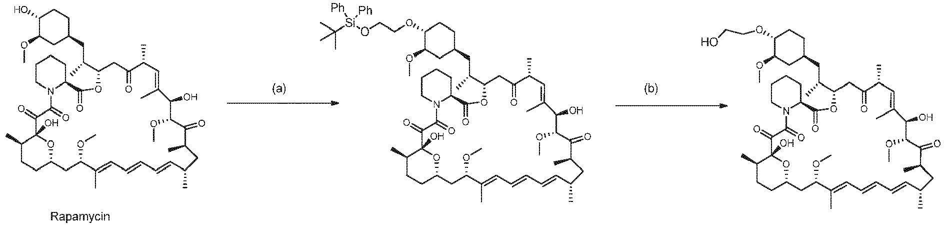

### Step (a)

To a solution of 2-((tert-butyldiphenylsilyl)oxy)ethanol (8.22 g, 27.3 mmol) in toluene (15 g) was added N,N-diisopropylpentan-3-amine (5.39 g, 31.4 mmol). The clear solution was then cooled to 0°C and trifluoromethanesulfonic acid anhydride (7.3 g, 26.0 mmol) was added dropwise such that the temperature was maintained between (-2°C - 2°C). Following the addition a further portion of toluene (5 g) was used for washing.

After 1.5 h N,N-diisopropylpentan-3-amine (5.39 g, 31.4 mmol) was added followed by toluene (2.5 g) and Rapamycin (10.0 g, 10.9 mmol) washing with toluene (12.5 g). The reaction was then heated to 45 °C and allowed to stir at this temperature for 21 h at which point less than 5 Area% Rapamycin was remaining according to HPLC analysis. The reaction was cooled to ambient temperature and pyridine (1.0 mL) was then added to quench the reaction which was stirred for a further 30 minutes. The reaction was filtered and diluted with isopropyl acetate. The organic solution was washed with 1M citric acid solution, 10% sodium bicarbonate solution followed by water, dried (MgSO₄) and concentrated in vacuo. The reaction mixture was divided into two portions, one of which was purified using flash column chromatography (0-100% ethyl acetate in heptanes) affording a yellow oil ca. 5.7 g.

### Step (b)

Pyridine (12.2 g, 154.4 mmol) was cooled to 0°C and then a solution of HF*Pyridine solution 65% (5.5 g) was added dropwise over 30 minutes. The resulting solution was then allowed to warm to ambient temperature and then the silyl-protected everolimus derivative (5.7 g) was added dropwise as a solution in THF (120 mL). The yellow solution was heated to 40 °C for 2 h and then allowed to cool to ambient temperature and diluted with isopropylacetate (150 g). The reaction was then added slowly to an 8% aqueous solution of sodium bicarbonate (500 g) and further diluted with isopropylacetate (250 g). The organic phase was then separated and washed with saturated aqueous sodium chloride solution, dried (MgSO₄) and concentrated in vacuo. The residue was diluted with isopropylacetate, butylhydroxytoluol (BHT; 0.2% m/m) was added, and the yield of everolimus determined by HPLC analysis against an external standard (3.14 g, 60.0% from Rapamycin).This material was then recrystallized from ethyl acetate/heptanes to afford Everolimus of high purity (2.05 g, 65%).

### Example 6 (Trityl protecting group)

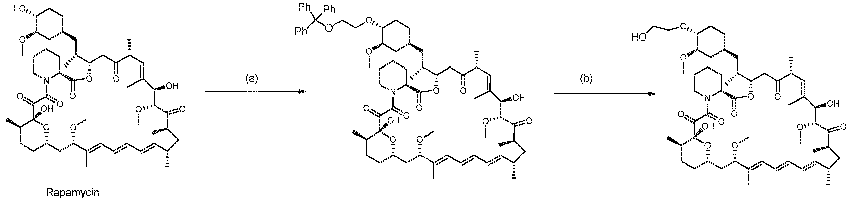

### Step (a)

To a solution of 2-(trityloxy)ethanol (10.7 g, 35.2 mmol) in toluene (42.8 g) was added N,N-diisopropylpentan-3-amine (7.0 g, 40.4 mmol). The clear solution was then cooled to 0°C and trifluoromethanesulfonic acid anhydride (10.0 g, 35.2 mmol) was added dropwise such that the temperature was maintained between (-2°C - 2°C).

After 2 h N,N-diisopropylpentan-3-amine (7.0 g, 40.4 mmol) was added to the reaction mixture followed by toluene (14.4 g) and Rapamycin (8.24 g, 8.79 mmol) washing with toluene (2.4 g). The reaction was then heated to 40 °C and allowed to stir at this temperature for 21 h at which point less than 5 Area% Rapamycin was remaining according to HPLC analysis. The reaction was cooled to ambient temperature and pyridine (0.8 mL) was then added to quench the reaction which was stirred for a further 30 minutes. The reaction was diluted with isopropyl acetate. The organic solution was washed with 1M citric acid solution, 10% sodium bicarbonate solution followed by water, dried (MgSO₄) and concentrated in vacuo. The reaction mixture was purified using flash column chromatography (ethyl acetate in heptanes) affording a yellow oil (6.85 g, 65% yield).

### Step (b)

The trityl-protected everolimus derivative (5.0 g, 4.165 mmol) was then dissolved in hexafluoroisopropanol and heated to 58 °C for 3.5 h. The reaction was then allowed to cool to ambient temperature and was diluted with ethyl acetate (50 mL) and concentrated in vacuo. This dilution/concentration procedure was repeated once more and then the crude product was filtered over silica gel (25 g) eluting with heptane/ethyl acetate. Recrystallization from heptanes/ethyl acetate afforded the desired product as white crystals (1.60 g, 40.1%). HPLC analysis of the mother liquor against an external standard indicated that a further 12% yield Everolimus was contained therein.

### Example 7

### Comparison of an effect that the base selection has on reaction yield

### Step 1:

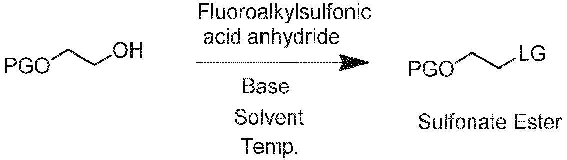

### Step 2:

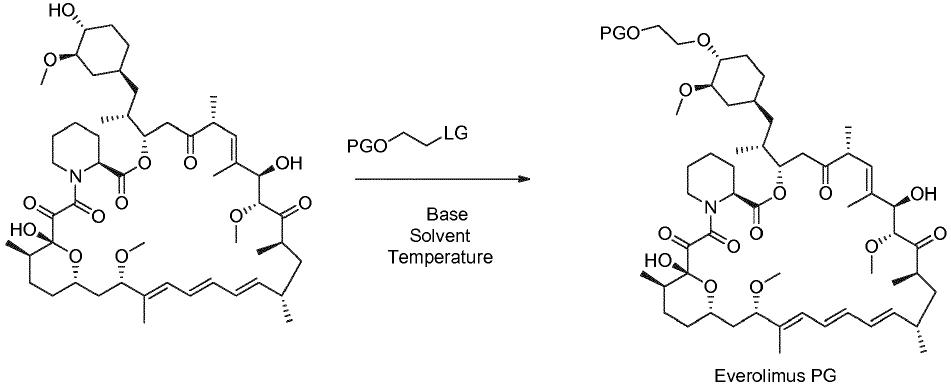

### Bases:

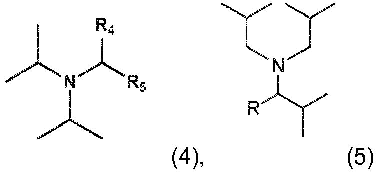

| Entry | PG | Base (Step 1) | Sulfonate Ester Eq. | Base (Step 2) | T [°C] | HPLC Area Everolimus -PG [%] | Yield* Everolimus |
|---|---|---|---|---|---|---|---|
| 1 | TBS | DIPEA | 4 | R4 = R5 = ethyl | 40 | 77.0 | 57% |
| 2 | TBS | DIPEA | 3.5 | R4 = R5 = ethyl | 50 | 76.0 | |
| 3 | TBS | R4 = R5 = ethyl | 3.5 | R4 = R5 = ethyl | 50 | 72.5 | |
| 4 | TBS | DIPEA | 5 | R: iPr | 50 | 79% | 51% |
| 5 | TBS | DIPEA | 5 | R: H | 50 | 75% | |
| 6 | TBS | DIPEA | 3.5 | R: H | 50 | 73.0 | 37% |
| 7 | TBS | R: H | 3.5 | R: H | 50 | 71.6 | 45% |
| 8 | TBS | DIPEA | 3.5 | R4 = R5 = butyl | 50 | 72.4 | |
| 9 | TBDPS | R4 = R5 = butyl | 4 | R4 = R5 = butyl | 40 | 76.5 | 61% |
| 10 | TBDPS | R4 = R5 = ethyl | 4 | R4 = R5 = ethyl | 40 | 79.1 | 65% |
| 11 | TBDPS | R4 = R5 = ethyl | 2.5 | R4 = R5 = ethyl | 40 | 78.1 | 67% |
| 12 | TBDPS | R: H | 4 | R: H | 50 | 71.9 | 47% |
| 13 | TBDPS | DIPEA | 6 | DIPEA | 50 | 74.0 | 50% |
| 14 | TBDPS | R4 = R5 = ethyl | 4* | R4 = R5 = ethyl | 30 | 81% | |
| 15 | Trityl | R4 = R5 = ethyl | 4 | R4 = R5 = ethyl | 40 | 75.5 | - |

| | | | | | | | |
|---|---|---|---|---|---|---|---|
| LG = trifluoromethanesulfonate; * Nonaflate (nonafluorobutanesulfonate) used in place of trifluoromethanesulfonate PG = protecting group; TBS = tert-butyldimethylsilyl, TBDPS = tert-butyldiphenylsilyl, TIPS = triisopropylsilyl DIPEA = N,N-diisopropylethylamine | | | | | | | |

The reactions were done as described in the specification with various combinations of bases, protecting groups and molar ratios of alkyl fluoroalkyl sulfonate (i.e. sulfonate ester)/rapamycin. The solvent used was always toluene. In step 1 the alkyl fluoroalkyl sulfonate was prepared. In step 2, rapamycin was alkylated to prepare protected everolimus.

The column "HPLC Area Everolimus-PG" shows the levels of the protected everolimus obtained. Subsequently, the protecting group was cleaved to obtain everolimus. The column "Yield* Everolimus Crude" shows the overall reaction yield. The R, R4 and R5 refer to the substituents in bases of compounds of formula (4) and (5). For example, if R4 = R5 = ethyl, it means that the base used was N,N-diisopropylpentan-3-amine. It is important to note that all reactions were led to more than 95% conversion (less than 5% rapamycin left).

HPLC Method Area % 3: Macherey-Nagel CC 250/4 Nucleosil 120-3 C18 Cat.No.: 721666.40, Mobile Phase: 80:20 methanol:water to 100:0 methanol:water over 35 minutes, Flow rate 1 mL/min, detection wavelength = 275 nm.

* Yield determined by HPLC analysis against an external standard of pure Everolimus HPLC Method Yield Determination: Atlantis-dC18, 3.0 µm, length 150 mm, internal diameter 3.0 mm (Waters no. 186001307) or equivalent column, mobile phase Ammonium acetate reagent + water + methanol + acetonitrile (160 + 160 + 320 + 360) (V/V/V/V), Flow rate 1.2 ml/min
Detection wavelength = 278 nm, run time = 25 minutes.

The results show that tris(2-methylpropyl)amine achieves a substantially lower yield of Everolimus Crude (Entry 12, 47%) in comparison to two of the bases covered by the present disclosure (Entry 9, 65% and Entry 10, 61%). Furthermore, the use of such hindered bases as described herein allows the use of a lower number of equivalents of the alkyl fluoroalkyl sulfonate (Entry 11, 2.5 equivalents of triflate used). The reaction described in Entry 12 using tris(2-methylpropyl)amine as base with 4 equivalents of triflate was much slower at 40 °C than the analogous reaction using N,N-diisopropylpentan-3-amine and it is for this reason that the reaction had to be performed at 50 °C to allow conversion >95% to be obtained. For clarity, if the reaction in the presence of tris(2-methylpropyl)amine had been left to run at 40 °C, the yield measured at the same time points would be even lower compared to yield obtained in the presence of bases as defined in the present disclosure. The same observation was made with N,N-diisopropylethylamine as base (Entry 13). In order to drive the reaction to completion a temperature of 50 °C and a total of 6 equivalents of the alkyl fluoroalkyl sulfonate were required. This procedure also provided the product in a yield of 50%, significantly lower than that obtained using two of the bases disclosed by the present disclosure (Entries 9 and 10).Therefore, the bases to be used according to the present disclosure allowed the process to achieve higher yield even at milder conditions (i.e. lower reaction temperature). In addition, smaller molar excess of alkyl fluoroalkyl sulfonates were required to achieve better yield.

## Claims

1. A process for preparing a chemical compound comprising reacting rapamycin with an alkyl fluoroalkyl sulfonate, wherein the alkyl part of the alkyl fluoroalkyl sulfonate can be further substituted; in the presence of a base, wherein the base is of formula NR1R2R3:
• R1 and R2 are independently 2-methylpropyl or isopropyl; and
• R3 is -CH(R4)(R5), wherein R4 and R5 are identical or different alkyls that are optionally connected to form a ring,
and wherein the alkyl is a straight or branched carbon chain of C2-C10-alkyl.

2. A process for preparing a chemical compound comprising reacting rapamycin with an alkyl fluoroalkyl sulfonate in the presence of a base according to claim 1, wherein the alkyl is a C2-C5-alkyl.

3. A process for preparing a chemical compound comprising reacting rapamycin with an alkyl fluoroalkyl sulfonate according to claim 1 or claim 2, wherein the fluoroalkyl sulfonate part of the alkyl fluoroalkyl sulfonate comprises C1-C4 alkyl substituted by at least one fluoride, preferably the alkyl fluoroalkyl sulfonate being alkyl trifluoromethylsulfonate, alkyl trifluoroethylsulfonate or alkyl nonafluorobutylsulfonate, especially is alkyl trifluoromethanesulfonate.

4. The process for preparing a chemical compound according to any one of claims 1 to 3, wherein the alkyl part of the alkyl fluoroalkyl sulfonate is substituted with a functional group, wherein the functional group is protected with a protecting group.

5. The process for preparing a chemical compound according to claim 4, further comprising a step of removing the protecting group.

6. A process for preparing an alkyl fluoroalkyl sulfonate, wherein the alkyl part of the alkyl fluoroalkyl sulfonate can be further substituted; by reacting an alcohol with a fluoroalkylsulfonic acid anhydride in the presence of a base, wherein the base is of formula NR1R2R3:
• R1 and R2 are independently 2-methylpropyl or isopropyl; and
• R3 is -CH(R4)(R5), wherein R4 and R5 are identical or different alkyls that are optionally connected to form a ring,
and wherein the alkyl is a straight or branched carbon chain of C2-C10-alkyl.

7. The process for preparing an alkyl fluoroalkyl sulfonate by reacting an alcohol with a fluoroalkylsulfonic acid anhydride in the presence of a base according to claim 6, wherein the alkyl is a C2-C5-alkyl.

8. A process for preparing an alkyl fluoroalkyl sulfonate according to claim 6 or claim 7, wherein a compound of formula (2) 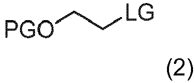 is prepared, PG being a protecting group and LG being fluoroalkyl sulfonate; the process comprising the step of reacting a compound of formula (3) 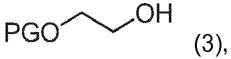 PG being a protecting group; with fluoroalkylsulfonic acid anhydride.

9. The process for preparing a chemical compound according to any one of claims 1 to 5, the process comprising the steps:
(a) reacting rapamycin with the compound of formula (2) in the presence of the base 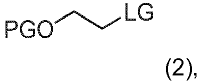 wherein
• PG is protecting group; and
• LG is fluoroalkyl sulfonate,
(b) removing the protecting group to obtain everolimus.

10. The process for preparing a chemical compound according to claim 9, wherein the LG is trifluoromethylsulfonate, trifluoroethylsulfonate or nonafluorobutylsulfonate.

11. Use of a base having formula NR1R2R3 in preparing everolimus, wherein:
• R1 and R2 are independently 2-methylpropyl or isopropyl; and
• R3 is -CH(R4)(R5), wherein R4 and R5 are identical or different alkyls that are optionally connected to form a ring;
in a chemical reaction comprising an alkyl fluoroalkyl sulfonate,
and wherein the alkyl is a straight or branched carbon chain of C2-C10-alkyl.

12. The use of a base having formula NR1R2R3 in preparing everolimus according to claim 11, wherein the alkyl is a C2-C5-alkyl.

13. The process according to any one of claims 1 to 10, or use according to claim 11 or claim 12, wherein the base has formula (4) 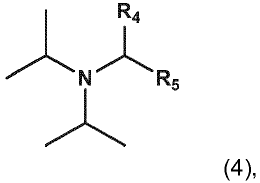 wherein R4 and R5 are identical or different alkyls that are optionally connected to form a ring;
or formula (5) 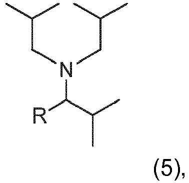 wherein R is an alkyl.

14. The process according to any one of claims 1 to 10, or use according to claim 11 or claim 12, wherein the base is N,N-diisopropylpentan-3-amine.

15. The process for preparing a chemical compound according to any one of claims 4 to 10, or use according to claim 11 or claim 12, wherein the base is N,N-diisopropylpentan-3-amine and the protecting group is selected from a group consisting of tert-butyldiphenylsilyl, dimethyl tert-hexylsilyl, tert-butyldimethylsilyl and trityl.

16. The process for preparing a chemical compound according to any one of claims 4 to 10, or use according to claim 11 or claim 12, wherein the base is N,N-diisopropylnonan-5-amine and the protecting group is selected from a group consisting of tert-butyldiphenylsilyl, dimethyl tert-hexylsilyl, tert-butyldimethylsilyl and trityl.

17. The process for preparing a chemical compound according to any one of claims 4 to 10, or use according to claim 11 or claim 12, wherein the base is N,N-diisobutyl-2,4-dimethylpentan-3-amine and the protecting group is selected from a group consisting of tert-butyldiphenylsilyl, dimethyl tert-hexylsilyl, tert-butyldimethylsilyl and trityl.

18. The process according to any one of claims 1 to 10, or use according to claim 11 or claim 12, wherein the base is N,N-diisopropylnonan-5-amine.

19. The process according to any one of claims 1 to 10, or use according to claim 11 or claim 12, wherein the base is N,N-diisobutyl-2,4-dimethylpentan-3-amine.

20. A process for preparing a pharmaceutical formulation, the process comprising the steps for preparing a chemical compound according to any one of claims 1 to 5, 9, 10, or 13 to 19, and mixing the chemical compound with at least one pharmaceutically acceptable excipient.

21. The process according to claim 20, wherein the chemical compound is everolimus.

## Patentansprüche

1. Verfahren zur Herstellung einer chemischen Verbindung, umfassend das Umsetzen von Rapamycin mit einem Alkylfluoralkylsulfonat, wobei der Alkylteil des Alkylfluoralkylsulfonats weiter substituiert sein kann; in Gegenwart einer Base, wobei die Base die Formel NR1R2R3 aufweist:
• R1 und R2 unabhängig für 2-Methylpropyl oder Isopropyl stehen und
• R3 für -CH(R4) (R5) steht, wobei R4 und R5 für gleiche oder verschiedene Alkylgruppen stehen, die gegebenenfalls zu einem Ring verbunden sind,
und wobei es sich bei dem Alkyl um eine gerade oder verzweigte Kohlenstoffkette von C2-C10-Alkyl handelt.

2. Verfahren zur Herstellung einer chemischen Verbindung, umfassend das Umsetzen von Rapamycin mit einem Alkylfluoralkylsulfonat nach Anspruch 1, wobei es sich bei dem Alkyl um ein C2-C5-Alkyl handelt.

3. Verfahren zur Herstellung einer chemischen Verbindung, umfassend das Umsetzen von Rapamycin mit einem Alkylfluoralkylsulfonat in Gegenwart einer Base nach Anspruch 1 oder Anspruch 2, wobei der Fluoralkylsulfonatteil des Alkylfluoralkylsulfonats C1-C4-Alkyl, das durch mindestens ein Fluorid substituiert ist, umfasst, wobei es sich bei dem Alkylfluoralkylsulfonat vorzugsweise um Alkyltrifluormethylsulfonat, Alkyltrifluorethylsulfonat oder Alkylnonafluorbutylsulfonat und insbesondere um Alkyltrifluormethylsulfonat handelt.

4. Verfahren zur Herstellung einer chemischen Verbindung nach einem der Ansprüche 1 bis 3, wobei der Alkylteil des Alkylfluoralkylsulfonats durch eine funktionelle Gruppe substituiert ist, wobei die funktionelle Gruppe durch eine Schutzgruppe geschützt ist.

5. Verfahren zur Herstellung einer chemischen Verbindung nach Anspruch 4, ferner umfassend einen Schritt des Abspaltens der Schutzgruppe.

6. Verfahren zur Herstellung eines Alkylfluoralkylsulfonats, wobei der Alkylteil das Alkylfluoralkylsulfonats weiter substituiert sein kann, durch Umsetzen eines Alkohols mit einem Fluoralkylsulfonsäureanhydrid in Gegenwart einer Base, wobei die Base die Formel NR1R2R3 aufweist:
• R1 und R2 unabhängig für 2-Methylpropyl oder Isopropyl stehen und
• R3 für -CH(R4) (R5) steht, wobei R4 und R5 für gleiche oder verschiedene Alkylgruppen stehen, die gegebenenfalls zu einem Ring verbunden sind,
und wobei es sich bei dem Alkyl um eine gerade oder verzweigte Kohlenstoffkette von C2-C10-Alkyl handelt.

7. Verfahren zur Herstellung eines Alkylfluoralkylsulfonats durch Umsetzen eines Alkohols mit einem Fluoralkylsulfonsäureanhydrid in Gegenwart einer Base nach Anspruch 6, wobei es sich bei dem Alkyl um ein C2-C5-Alkyl handelt.

8. Verfahren zur Herstellung eines Alkylfluoralkylsulfonats nach Anspruch 6 oder Anspruch 7, wobei eine Verbindung der Formel (2) 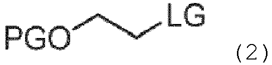 hergestellt wird, wobei PG für eine Schutzgruppe steht und LG für Fluoralkylsulfonat steht;
wobei das Verfahren den Schritt des Umsetzens einer Verbindung der Formel (3) 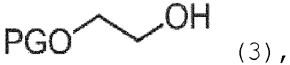 wobei PG für eine Schutzgruppe steht,
mit Fluoralkylsulfonsäureanhydrid umfasst.

9. Verfahren zur Herstellung einer chemischen Verbindung nach einem der Ansprüche 1 bis 5, wobei das Verfahren folgende Schritte umfasst:
(a) Umsetzen von Rapamycin mit der Verbindung der Formel (2) in Gegenwart der Base 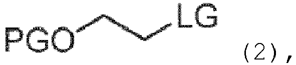 wobei
• PG für eine Schutzgruppe steht und
• LG für Fluoralkylsulfonat steht,
(b) Abspalten der Schutzgruppe zum Erhalt von Everolimus.

10. Verfahren zur Herstellung einer chemischen Verbindung nach Anspruch 9, wobei die LG für Trifluormethylsulfonat, Trifluorethylsulfonat oder Nonafluorbutylsulfonat steht.

11. Verwendung einer Base der Formel NR1R2R3 zur Herstellung von Everolimus, wobei:
• R1 und R2 unabhängig für 2-Methylpropyl oder Isopropyl stehen und
• R3 für -CH(R4) (R5) steht, wobei R4 und R5 für gleiche oder verschiedene Alkylgruppen stehen, die gegebenenfalls zu einem Ring verbunden sind, in einer chemischen Reaktion, die ein Alkylfluoralkylsulfonat umfasst, und wobei es sich bei dem Alkyl um eine gerade oder verzweigte Kohlenstoffkette von C2-C10-Alkyl handelt.

12. Verwendung einer Base der Formel NR1R2R3 bei der Herstellung von Everolimus nach Anspruch 11, wobei es sich bei dem Alkyl um ein C2-C5-Alkyl handelt.

13. Verfahren nach einem der Ansprüche 1 bis 10 oder Verwendung nach Anspruch 11 oder Anspruch 12, wobei die Base die Formel (4) 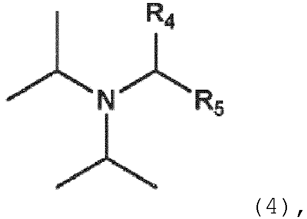 wobei R4 und R5 für gleiche oder verschiedene Alkylgruppen stehen, die gegebenenfalls zu einem Ring verbunden sind,
oder Formel (5) 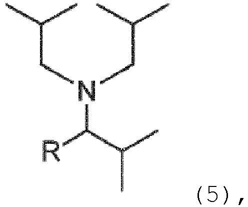 wobei R für ein Alkyl steht, aufweist.

14. Verfahren nach einem der Ansprüche 1 bis 10 oder Verwendung nach Anspruch 11 oder 12, wobei es sich bei der Base um N,N-Diisopropylpentan-3-amin handelt.

15. Verfahren zur Herstellung einer chemischen Verbindung nach einem der Ansprüche 4 bis 10 oder Verwendung nach Anspruch 11 oder 12, wobei es sich bei der Base um N,N-Diisopropylpentan-3-amin handelt und die Schutzgruppe aus der Gruppe bestehend aus tert-Butyldiphenylsilyl, Dimethyltert-hexylsilyl, tert-Butyldimethylsilyl und Trityl ausgewählt wird.

16. Verfahren zur Herstellung einer chemischen Verbindung nach einem der Ansprüche 4 bis 10 oder Verwendung nach Anspruch 11 oder 12, wobei es sich bei der Base um N,N-Diisopropylnonan-5-amin handelt und die Schutzgruppe aus der Gruppe bestehend aus tert-Butyldiphenylsilyl, Dimethyltert-hexylsilyl, tert-Butyldimethylsilyl und Trityl ausgewählt wird.

17. Verfahren zur Herstellung einer chemischen Verbindung nach einem der Ansprüche 4 bis 10 oder Verwendung nach Anspruch 11 oder 12, wobei es sich bei der Base um N,N-Diisobutyl-2,4-dimethylpentan-3-amin handelt und die Schutzgruppe aus der Gruppe bestehend aus tert-Butyldiphenylsilyl, Dimethyltert-hexylsilyl, tert-Butyldimethylsilyl und Trityl ausgewählt wird.

18. Verfahren nach einem der Ansprüche 1 bis 10 oder Verwendung nach Anspruch 11 oder Anspruch 12, wobei es sich bei der Base um N,N-Diisopropylnonan-5-amin handelt.

19. Verfahren nach einem der Ansprüche 1 bis 10 oder Verwendung nach Anspruch 11 oder Anspruch 12, wobei es sich bei der Base um N,N-Diisobutyl-2,4-dimethylpentan-3-amin handelt.

20. Verfahren zur Herstellung einer pharmazeutischen Formulierung, bei dem man eine chemische Verbindung nach einem der Ansprüche 1 bis 5, 9, 10 oder 13 bis 19 herstellt und die chemische Verbindung mit mindestens einem pharmazeutisch unbedenklichen Hilfsstoff mischt.

21. Verfahren nach Anspruch 20, wobei es sich bei der chemischen Verbindung um Everolimus handelt.

## Revendications

1. Procédé de préparation d'un composé chimique comprenant la réaction de rapamycine avec un fluoroalkylsulfonate d'alkyle, dans lequel la partie alkyle du fluoroalkylsulfonate d'alkyle peut en outre être substituée ; en présence d'une base, la base étant de formule NR1R2R3 :
• R1 et R2 sont indépendamment 2-méthylpropyle ou isopropyle ; et
• R3 est -CH(R4)(R5), où R4 et R5 sont des alkyles identiques ou différents qui sont facultativement reliés de façon à former un cycle,
et dans lequel l'alkyle est une chaîne carbonée linéaire ou ramifiée d'alkyle en C2-C10.

2. Procédé de préparation d'un composé chimique comprenant la réaction de rapamycine avec un fluoroalkylsulfonate d'alkyle en présence d'une base selon la revendication 1, dans lequel l'alkyle est un alkyle en C2-C5.

3. Procédé de préparation d'un composé chimique comprenant la réaction de rapamycine avec un fluoroalkylsulfonate d'alkyle selon la revendication 1 ou la revendication 2, dans lequel la partie fluoroalkylsulfonate du fluoroalkylsulfonate d'alkyle comprend un alkyle en C1-C4 substitué par au moins un fluorure, de préférence le fluoroalkylsulfonate d'alkyle étant un trifluorométhylsulfonate d'alkyle, un trifluoroéthylsulfonate d'alkyle ou un nonafluorobutylsulfonate d'alkyle, en particulier, est un trifluorométhanesulfonate d'alkyle.

4. Procédé de préparation d'un composé chimique selon l'une quelconque des revendications 1 à 3, dans lequel la partie alkyle du fluoroalkylsulfonate d'alkyle est substituée par un groupe fonctionnel, dans lequel le groupe fonctionnel est protégé avec un groupe protecteur.

5. Procédé de préparation d'un composé chimique selon la revendication 4, comprenant en outre une étape de retrait du groupe protecteur.

6. Procédé de préparation d'un fluoroalkylsulfonate d'alkyle, dans lequel la partie alkyle du fluoroalkylsulfonate d'alkyle peut être substituée plus avant ; par réaction d'un alcool avec un anhydride d'acide fluoroalkylsulfonique en présence d'une base, dans lequel la base est de formule NR1R2R3 :
• R1 et R2 sont indépendamment 2-méthylpropyle ou isopropyle ; et
• R3 est -CH(R4)(R5), dans lequel R4 et R5 sont des alkyles identiques ou différents qui sont facultativement reliés pour former un cycle,
et dans lequel l'alkyle est une chaîne carbonée linéaire ou ramifiée d'alkyle en C2-C10.

7. Procédé de préparation d'un fluoroalkylsulfonate d'alkyle par réaction d'un alcool avec un anhydride d'acide fluoroalkylsulfonique en présence d'une base selon la revendication 6, dans lequel l'alkyle est un alkyle en C2-C5.

8. Procédé de préparation d'un fluoroalkylsulfonate d'alkyle selon la revendication 6 ou la revendication 7, dans lequel un composé de formule (2) 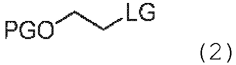 est préparé, PG étant un groupe protecteur et LG étant un fluoroalkylsulfonate ;
le procédé comprenant l'étape de réaction d'un composé de formule (3) 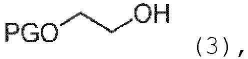 PG étant un groupe protecteur ;
avec l'anhydride d'acide fluoroalkylsulfonique.

9. Procédé de préparation d'un composé chimique selon l'une quelconque des revendications 1 à 5, le procédé comprenant les étapes de :
(a) réaction de rapamycine avec le composé de formule (2) en présence de la base 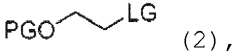 dans lequel
• PG est un groupe protecteur ; et
• LG est un fluoroalkylsulfonate,
(b) retrait du groupe protecteur pour obtenir l'évérolimus.

10. Procédé de préparation d'un composé chimique selon la revendication 9, dans lequel LG est trifluorométhylsulfonate, trifluoroéthylsulfonate ou nonafluorobutylsulfonate.

11. Utilisation d'une base ayant la formule NR1R2R3 dans la préparation d'évérolimus, dans laquelle :
• R1 et R2 sont indépendamment 2-méthylpropyle ou isopropyle ; et
• R3 est -CH(R4)(R5), où R4 et R5 sont des alkyles identiques ou différents qui sont facultativement reliés de façon à former un cycle ;
dans une réaction chimique comprenant un fluoroalkylsulfonate d'alkyle,
et où l'alkyle est une chaîne carbonée linéaire ou ramifiée d'alkyle en C2-C10.

12. Utilisation d'une base ayant la formule NR1R2R3 dans la préparation d'évérolimus selon la revendication 11, dans laquelle l'alkyle est un alkyle en C2-C5.

13. Procédé selon l'une quelconque des revendications 1 à 10, ou utilisation selon la revendication 11 ou la revendication 12, dans lequel la base a la formule (4) 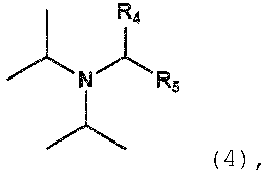 dans laquelle R4 et R5 sont des alkyles identiques ou différents qui sont facultativement reliés pour former un cycle ;
ou la formule (5) 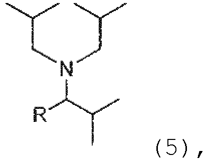 dans laquelle R est un alkyle.

14. Procédé selon l'une quelconque des revendications 1 à 10, ou utilisation selon la revendication 11 ou la revendication 12, dans lequel la base est la N,N-diisopropylpentan-3-amine.

15. Procédé de préparation d'un composé chimique selon l'une quelconque des revendications 4 à 10, ou utilisation selon la revendication 11 ou la revendication 12, dans lequel la base est la N,N-diisopropylpentan-3-amine et le groupe protecteur est choisi dans un groupe constitué de tert-butyldiphénylsilyle, diméthyl-tert-hexylsilyle, tert-butyldiméthylsilyle et trityle.

16. Procédé de préparation d'un composé chimique selon l'une quelconque des revendications 4 à 10, ou utilisation selon la revendication 11 ou la revendication 12, dans lequel la base est la N,N-diisopropylnonan-5-amine et le groupe protecteur est choisi dans un groupe constitué des tert-butyldiphénylsilyle, diméthyl-tert-hexylsilyle, tert-butyldiméthylsilyle et trityle.

17. Procédé de préparation d'un composé chimique selon l'une quelconque des revendications 4 à 10, ou utilisation selon la revendication 11 ou la revendication 12, dans lequel la base est la N,N-diisobutyl-2,4-diméthylpentan-3-amine et le groupe protecteur est choisi dans un groupe constitué de tert-butyldiphénylsilyle, diméthyl-tert-hexylsilyle, tert-butyldiméthylsilyle et trityle.

18. Procédé selon l'une quelconque des revendications 1 à 10, ou utilisation selon la revendication 11 ou la revendication 12, dans lequel la base est la N,N-diisopropylnonan-5-amine.

19. Procédé selon l'une quelconque des revendications 1 à 10, ou utilisation selon la revendication 11 ou la revendication 12, dans lequel la base est la N,N-diisobutyl-2,4-diméthylpentan-3-amine.

20. Procédé de préparation d'une formulation pharmaceutique, le procédé comprenant les étapes de préparation d'un composé chimique selon l'une quelconque des revendications 1 à 5, 9, 10, ou 13 à 19, et de mélange du composé chimique avec au moins un excipient pharmaceutiquement acceptable.

21. Procédé selon la revendication 20, dans lequel le composé chimique est l'évérolimus.
